# FASCICULE DE BREVET EUROPEEN

(11) **EP 1 676 606 B1**
(45) Date de publication et mention de la délivrance du brevet: **25.04.2007**
(21) Numéro de dépôt: 05292099.8
(22) Date de dépôt: 10.10.2005
(51) Int. Cl.: A61Q 19/06, A61K 8/49, A61K 8/87, A61K 8/898, A61K 8/97

(54) **Composition amincissante comprenant une base xanthique, un diméthicone copolyol et une poudre de polyuréthane**
Auf Xanthin basierende, schlankmachende Zusammensetzung enthaltend ein Dimethiconcopolyol und ein Polyurethanpulver
Slimming composition comprising a xanthin base, a dimethicone copolyol and a polyurethane powder

(30) Priorité: 17.12.2004 FR 0453051
(43) Date de publication de la demande: 05.07.2006
(73) Titulaire: L'ORÉAL, 75008 Paris (FR)
(72) Inventeur: Fonolla Moreno, Angeles, 75013 Paris (FR); Piot, Bertrand, 75009 Paris (FR)
(74) Mandataire: Kromer, Christophe

(56) Documents cités:
- FR-A- 2 845 912
- US-A- 5 194 259
- US-A1- 2003 180 242
- US-A1- 2004 223 935

## Description

La présente invention a pour objet une composition cosmétique comprenant un diméthicone copolyol, une base xanthique et une poudre de polyuréthane. L'invention a également pour objet un procédé cosmétique pour lutter contre la cellulite et/ou la peau d'orange et/ou amincir la silhouette, comprenant l'application topique de la composition sur la peau.

L'adiposité (ou excès de graisse dans le tissu cellulaire sous-cutané) peut avoir de nombreuses causes plus ou moins complexes.

Certaines cellules de la peau, appelées adipocytes, contiennent des quantités variables de graisses sous la forme de triglycérides, ces triglycérides étant synthétisés *in vivo* par les adipocytes eux-mêmes, selon des réactions de type enzymatique (lipogénèse), à partir des acides gras libres et du glucose (après dégradation de ce dernier en glycérol) contenus dans l'organisme et apportés à celui-ci par l'intermédiaire de certains aliments. Or, parallèlement, les triglycérides ainsi formés, puis stockés, dans les cellules adipocytaires peuvent également se redécomposer, toujours sous l'action d'enzymes spécifiques (lipolyse) contenues dans ces mêmes cellules, en libérant cette fois des acides gras d'une part et du glycérol et/ou des mono- et/ou des di-esters du glycérol d'autre part. Les acides gras ainsi relargués peuvent alors soit diffuser dans l'organisme pour y être consommés ou transformés de différentes façons, soit être recaptés (aussitôt ou un peu plus tard) par les adipocytes pour générer à nouveau des triglycérides par lipogénèse.

Si, pour des raisons diverses (nourriture trop riche, inactivité, vieillissement et autres), un déséquilibre substantiel s'installe dans l'organisme entre la lipogénèse et la lipolyse, c'est à dire plus précisément si les quantités de graisses formées par lipogénèse deviennent notablement et constamment supérieures à celles qui sont éliminées par lipolyse, il se produit alors dans les adipocytes une accumulation de triglycérides qui, si elle devient excessive, peut se traduire progressivement par l'apparition d'une peau épaisse, à surface souvent irrégulière ("peau d'orange") et de consistance plus ou moins flasque ou gélatineuse, donnant finalement à la silhouette un aspect général disgracieux pouvant évoluer entre la simple surcharge locale (lipodismorphie) et la formation de cellulite, en passant par l'embonpoint certain, et enfin la réelle obésité.

Or, compte tenu notamment du profond inconfort tant physique qu'esthétique, et parfois psychologique, qu'elles occasionnent auprès des individus qui en sont atteints, en particulier chez les femmes, l'adiposité et la cellulite constituent de nos jours une affection de moins en moins bien supportée ou acceptée.

Des solutions ont donc été proposées dans l'art antérieur pour intervenir sur le métabolisme des acides gras qui est, comme on l'a vu, l'une des cibles privilégiées dans le contrôle de cette surcharge lipidique adipocytaire.

Celui-ci peut être modulé:
- soit par blocage du transport du glucose à l'intérieur de l'adipocyte qui conduit, comme on l'a vu, à une diminution des acides gras entrant dans l'adipocyte,
- soit par inhibition de la lipoprotéine lipase,
- soit par activation de la triglycéride lipase (ou lipase hormonosensible), généralement en stimulant l'AMP cyclique, par exemple par activation de l'adényl cyclase, ou en provoquant son accumulation par inhibition de la phosphodiestérase.

D'autres voies biologiques ont été explorées pour agir sur le mécanisme de la lipogénèse et/ou de la lipolyse. Il a ainsi été proposé d'utiliser des antagonistes des récepteurs du neuropeptide Y (NPY), qui est un neuromédiateur intervenant dans un certain nombre de processus physiologiques et dont l'implication dans la régularisation de la lipolyse a pu être démontrée (P. Valet, J. Clin. Invest., 1990, 85, 291-295). On peut également utiliser des antagonistes des récepteurs α_{z} ou encore des agonistes des récepteurs β3-adrénergiques.

Les compositions cosmétiques proposées jusqu'à présent en vue de traiter l'adiposité contiennent donc des composés dits amincissants qui agissent sur un ou plusieurs des mécanismes mentionnés précédemment. Parmi ceux-ci, on peut plus particulièrement citer les bases xanthiques (i.e. des dérivés de la xanthine), telles que la théophylline, la caféine, la théobromine.

Toutefois, les bases xanthiques comme la caféine ont l'inconvénient de conférer aux compositions amincissantes de mauvaises propriétés cosmétiques ressenties par l'utilisatrice après l'application de la composition sur la peau. La peau traitée présente un effet collant, une sensation de tiraillement, un manque de douceur : ces propriétés néfastes ne satisfont pas l'utilisatrice.

Le but de la présente invention est donc de disposer d'une composition cosmétique à action amincissante contenant une base xanthique et présentant de bonnes propriétés cosmétiques après l'application de la composition sur la peau, notamment de bonnes propriétés de non collant et de douceur.

Les inventeurs ont découvert qu'une telle composition peut être obtenue en utilisant avec la base xanthique une poudre de polyuréthane et un diméthicone copolyol non ionique.

La composition appliquée sur la peau ne présente pas d'effet collant et confère une sensation de douceur agréable sur la peau, sans effet de tiraillement. En outre, la composition présente une bonne efficacité amincissante.

De façon plus précise, l'invention a pour objet une composition comprenant, dans un milieu physiologiquement acceptable, au moins une base xanthique ou un extrait végétal en contenant, au moins une poudre de polyuréthane et au moins un diméthicone copolyol non ionique.

L'invention a également pour objet un procédé cosmétique pour lutter contre la cellulite et/ou la peau d'orange et/ou amincir la silhouette, comprenant l'application sur la peau d'une composition telle que définie précédemment.

La composition selon l'invention est généralement adaptée à une application topique sur la peau et comprend donc généralement un milieu physiologiquement acceptable, c'est-à-dire compatible avec la peau. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention comprend au moins une base xanthique ou un extrait végétal en contenant.

Parmi les bases xanthiques utilisables selon l'invention, on peut citer : la caféine, la théophylline, la théobromine, l'acéfylline, le nicotinate de xanthinol, la diniprophylline, la diprophylline, l'étamiphylline et ses dérivés, l'étophylline, la proxyphylline, la pentophylline, la propentophylline, la pyridophylline et la bamiphylline, sans que cette liste ne soit limitative.

On préfère en particulier utiliser la caféine, la théophylline, la théobromine et l'acéfylline, et plus particulièrement la caféine. Ces bases xanthiques sont connues comme inhibiteurs de phosphodiestérase, qui est l'enzyme responsable de la dégradation de l'AMPc. En augmentant le taux intra-cellulaire d'AMPc, ces bases xanthiques favorisent l'activité lipolytique et constituent donc des actifs amincissants de premier ordre.

Comme exemples d'extraits végétaux renfermant des bases xanthiques, on peut notamment citer les extraits de thé, de café, de guarana, de maté et de cola, sans que cette liste ne soit limitative.

La base xanthique peut être présente dans la composition selon l'invention en une teneur allant de 0,01 % à 10% en poids, par rapport au poids total de la composition, de préférence allant de 0,1 % à 7% en poids, et préférentiellement allant de 0,1 % à 3 % en poids, notamment allant de 1 % à 3 % en poids.

La composition selon l'invention contient une poudre de polyuréthane. En particulier, la poudre de polyuréthane n'est pas filmogène, c'est-à-dire qu'elle ne forme pas un film continu lorsqu'elle est déposée sur un support tel que la peau.

Avantageusement, la poudre de polyuréthane est une poudre de copolymère de diisocyanate d'hexaméthylène et de triméthylol hexyl lactone. Une telle poudre de polyuréthane est notamment vendue sous les dénominations « PLASTIC POWDER D-400 », « PLASTIC POWDER D-800 » par la société TOSHIKI.

Comme autre poudre de polyuréthane, on peut utiliser celle vendue sous la dénomination « PLASTIC POWDER CS-400 » par la société TOSHIKI.

La poudre de polyuréthane peut être présente en une teneur allant de 0,5 % à 10 % en poids, par rapport au poids total de la composition, et de préférence allant de 0,5 % à 6 % en poids, et préférentiellement allant de 0,5 % à 3 % en poids.

La composition selon l'invention comprend un diméthicone copolyol non ionique.

Le diméthicone copolyol non ionique est un polydiméthyl méthyl siloxane oxypropyléné et/ou oxyéthyléné qui ne contient pas de groupement ionique (tels que des groupements anionique, cationique, amphotère).

Des diméthicone copolyols non ioniques sont notamment décrit dans le brevet US-A-4268499.

Un forme de diméthicone copolyol particulièrement préférée est celui vendu sous la dénomination DOW CORNING 5225C par la société DOW CORNING.

Le diméthicone copolyol non ionique peut être présent dans la composition selon l'invention en une teneur allant de 0,05 % à 5 % en poids, par rapport au poids total de la composition, de préférence allant de 0,1 % à 3 % en poids, et préférentiellement allant de 0,1 % à 2 % en poids.

La composition selon l'invention peut comprendre avantageusement une phase aqueuse.

La composition peut comprendre de l'eau en une teneur allant de 30 % à 80 % en poids, par rapport au poids total de la composition, de préférence allant de 40 % à 70 % en poids, et préférentiellement allant de 45 % à 65 % en poids.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut comprendre au moins un monoalcool ayant de 2 à 6 atomes de carbone tels que l'éthanol, l'isopropanol, notamment en une teneur allant de 1 % à 30 % en poids, par rapport au poids total de la composition, et de préférence allant de 5 % à 20 % en poids.

La phase aqueuse peut comprendre en outre de l'acide salicylique ou l'un de ses dérivés comme l'acide n-octanoyle, notamment présent en une teneur allant de 0,1 % à 5 % en poids, par rapport au poids total de la composition, de préférence allant de 0,1 % à 3 % en poids, et préférentiellement allant de 0,1 % à 2 % en poids.

La présence de monoalcool et d'acide salicylique dans la phase aqueuse permet de favoriser une bonne dissolution de la base xanthique dans la phase aqueuse et donc d'améliorer l'efficacité amincissante de la composition.

La composition selon l'invention peut se présenter sous toutes les formes galéniques normalement utilisées pour une application topique sur la peau, notamment sous forme d'une émulsion huile-dans-eau ou eau-dans-huile ou multiple, éventuellement gélifiée, d'une émulsion siliconée, d'une microémulsion ou nanoémulsion, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique. Avantageusement , la composition est sous forme d'émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les agents hydratants (tels que la glycérine, le propylène glycol, le butylène glycol, le pentylène glycol, l'hexylène glycol, le dipropylène glycol, le diéthylène glycol), les conservateurs, les antioxydants, les solvants, les parfums, les charges, les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition selon l'invention est une émulsion, la proportion de la phase grasse peut aller de 5 à 50 % en poids, et de préférence de 5 à 30 % en poids par rapport au poids total de la composition.

Comme matières grasses utilisables dans l'invention, on peut utiliser les huiles minérales, les huiles d'origine animale, les huiles de synthèse, les huiles siliconées et les huiles fluorées. On peut aussi utiliser comme matières grasses des acides gras, des cires et des gommes et en particulier les gommes de silicone.

Les émulsionnants et les co-émulsionnants éventuellement utilisés dans la composition sous forme d'émulsion sont choisis parmi ceux classiquement utilisés dans le domaine considéré. Ces émulsionnants et coémulsionnants sont de préférence présents, dans la composition, en une proportion allant de 0,3 à 20 % en poids, et de préférence de 0,5 à 5 % en poids par rapport au poids total de la composition. Comme émulsionnants et coémulsionnants utilisables dans l'invention, il est particulièrement avantageux d'utiliser les esters d'acide gras et de polyol tels que le stéarate de PEG-100, le stéarate de PEG-50 et le stéarate de PEG-40 ; le tristéarate de sorbitane, les stéarates de sorbitane oxyéthylénés disponibles sous les dénominations commerciales Tween^{®} 20 ou Tween^{®} 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs qui vont compléter l'action des actifs amincissants selon l'invention, on peut utiliser notamment :
- les actifs agissant sur la microcirculation (vasoprotecteurs ou vasodilatateurs), tels que les flavonoïdes, les extraits de Gingko biloba, les ruscogénines, les esculosides, l'escine extraite du marron d'Inde, les nicotinates, l'hespéridine méthyl chalcone, le ruscus ou petit houx, les huiles essentielles de lavande ou de romarin ;
- les actifs raffermissants et/ou anti-glycants (empêchant la fixation du sucre sur les fibres de collagène), tels que les extraits de Centella asiatica et de Siegesbeckia, qui stimulent la synthèse de collagène, le silicium, l'amadorine, la vitamine C et ses dérivés et le rétinol et ses dérivés ;
- et leurs mélanges.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

La composition définie précédemment peut être utilisée pour prévenir ou lutter contre la cellulite et/ou pour affiner la silhouette ou les contours du visage.

L'invention sera maintenant illustrée à l'aide des exemples non limitatifs suivants.

### Exemples 1 à 4 :

On a réalisé 3 compositions amincissantes selon l'invention (exemples 2 à 4) contenant un diméthicone copolyol non ionique et une poudre de polyuréthane, et une composition hors invention (exemple 1) contenant un diméthicone copolyol anionique et une poudre de silice.

On a appliqué la composition sur la peau et observé les propriétés cosmétiques obtenues.

Les teneurs sont exprimées en gramme.

| **COMPOSITION** | **Exemple 1*** | **Exemple 2** | **Exemple 3** | **Exemple 4** |
|---|---|---|---|---|
| **PHASE I** | | | | |
| Eau | qsp | qsp | qsp | qsp |
| Gélifiant acrylique(1) | 0.5 | 0.5 | | 0.2 |
| Gomme de Xanthane | 0.1 | 0.08 | | |
| AMPS (2) | | 0.1 | 1 | 0.6 |
| Glycérine | 4 | 4 | 5 | 4 |
| Propylène glycol | 3 | 3 | 3 | 3 |
| Caféine | 2.5 | 2.5 | 3 | 4 |
| Acide salicylique | 0.4 | 0.4 | 0.5 | 0.6 |
| | | | | |
| **PHASE II** | | | | |
| Diméthicone copolyol (3) | 0 | 2 | 3 | 5 |
| Huiles silicones volatiles | 9 | 9 | 7 | 5 |
| Pecosil PS-100 (4) | 1 | - | - | - |
| Gommes de silicone | 0.5 | 0.5 | 1 | 2 |
| Parfum | 0.4 | 0.4 | 0.5 | 0.5 |
| | | | | |
| **PHASE III** | | | | 1 |
| Ethanol | 12 | 12 | 16 | 18 |
| | | | | |
| **PHASE IV** | | | | |
| Poudre de polyuréthane (5) | | 1 | 0.8 | 2 |
| Silice | 1 | | | |
| | | | | |
| Propriétés cosmétiques | Pas d'effet doux. La peau accroche et tire. Sensation collante. | Effet doux et glissant, pas collant. | Peau très douce, pas d'effet collant. | Peau très douce, pas d'effet collant. |

| | | | | |
|---|---|---|---|---|
| * : exemple hors invention (1) Polymère carboxyvinylique vendu sous la dénomination « CARBOPOL 980 » par la société NOVEON. (2) Acide polyacrylamido propane sulfonique neutralisé partiellement à l'ammoniaque et réticulé, vendu sous la dénomination « HOSTACERIN AMPS » par la société CLARIANT. (3) Mélange de diméthicone copolyol/cyclopentadiméthylsiloxane/eau (10/88/2) vendu sous la dénomination « DOW CORNING 5225C FORMULATION AID » par la société DOW CORNING. (4) Polydimethylsiloxane oxyéthyléné à groupement phosphate vendu sous la dénomination « PECOSIL PS-100 » par la société PHOENIX CHEMICAL (5) Poudre de polyuréthane vendue sous la dénomination « PLASTIC POWDER D-400 » par la société TOSHIKI | | | | |

On a constaté que les compositions des exemples 2 à 4 selon l'invention, après application sur la peau, présentent une bonne douceur et ne sont pas collante au toucher tandis que la composition de l'exemple 1 hors invention confère un tiraillement à la peau (pas de douceur) et présente un aspect collant.

## Revendications

1. Composition comprenant, dans un milieu physiologiquement acceptable, au moins une base xanthique ou un extrait végétal en contenant, au moins une poudre de polyuréthane, et au moins un diméthicone copolyol non ionique.

2. Composition selon la revendication précédente, **caractérisée par le fait que** la base xanthique est choisie parmi : la caféine, la théophylline, la théobromine, l'acéfylline, le nicotinate de xanthinol, la diniprophylline, la diprophylline, l'étamiphylline et ses dérivés, l'étophylline, la proxyphylline, la pentophylline, la propentophylline, la pyridophylline et la bamiphylline.

3. Composition selon l'une quelconque des revendications précédentes, **caractérisée par le fait que** la base xanthique est choisie parmi la caféine, la théophylline, la théobromine et l'acéfylline.

4. Composition selon la revendication précédente, **caractérisée par le fait que** ledit extrait végétal est choisi parmi les extraits de thé, de café, de guarana, de maté et de cola.

5. Composition selon l'une quelconque des revendications précédentes, **caractérisée par le fait que** la base xanthique est présente en une teneur allant de 0,01 % à 10% en poids, par rapport au poids total de la composition, de préférence allant de 0,1 % à 7% en poids, et préférentiellement allant de 0,1 % à 3 % en poids.

6. Composition selon l'une quelconque des revendications précédentes, **caractérisée par le fait que** la poudre de polyuréthane n' est pas filmogène.

7. Composition selon l'une quelconque des revendications précédentes, **caractérisée par le fait que** la poudre de polyuréthane est une poudre de copolymère de diisocyanate d'hexaméthylène et de triméthylol hexyl lactone.

8. Composition selon l'une quelconque des revendications précédentes, **caractérisée par le fait qu'**elle comprend une poudre de polyuréthane en une teneur allant de 0,5 % à 10 % en poids, par rapport au poids total de la composition, et de préférence allant de 0,5 % à 6 % en poids, et préférentiellement allant de 0,5 % à 3 % en poids.

9. Composition selon l'une quelconque des revendications précédentes, **caractérisée par le fait que** le diméthicone copolyol non ionique est un polydiméthyl méthyl siloxane oxypropyléné et/ou oxyéthyléné ne contenant pas de groupement ionique.

10. Composition selon l'une quelconque des revendications précédentes, **caractérisée par le fait que** le diméthicone copolyol non ionique est présent en une teneur allant de 0,05 % à 5 % en poids, par rapport au poids total de la composition, de préférence allant de 0,1 % à 3 % en poids, et préférentiellement allant de 0,1 % à 2 % en poids.

11. Composition selon l'une quelconque des revendications précédentes, **caractérisée par le fait qu'**elle comprend une phase aqueuse.

12. Composition selon la revendication précédente, **caractérisée par le fait qu'**elle comprend de l'eau en une teneur allant de 30 % à 80 % en poids, par rapport au poids total de la composition, de préférence allant de 40 % à 70 % en poids, et préférentiellement allant de 45 % à 65 % en poids.

13. Composition selon l'une quelconque des revendications 11 ou 12, **caractérisée par le fait qu'**elle comprend un monoalcool ayant de 2 à 6 atomes de carbone.

14. Composition selon la revendication précédente, **caractérisée par le fait que** le monoalcool est choisi parmi l'éthanol, l'isopropanol.

15. Composition selon la revendication 13 ou 14, **caractérisée par le fait que** le monoalcool est présent en une teneur allant de 1 % à 30 % en poids, par rapport au poids total de la composition, et de préférence allant de 5 % à 20 % en poids.

16. Composition selon l'une quelconque des revendications 12 à 15, **caractérisée par le fait qu'**elle comprend de l'acide salicylique ou l'un de ses dérivés.

17. Composition selon la revendication précédente, **caractérisée par le fait que** l'acide salicylique ou ses dérivés est présent en une teneur allant de 0,1 % à 5 % en poids, par rapport au poids total de la composition, de préférence allant de 0,1 % à 3 % en poids, et préférentiellement allant de 0,1 % à 2 % en poids.

18. Composition selon l'une quelconque des revendications précédentes, **caractérisée par le fait qu'**elle se présente sous la forme d'émulsion huile-dans-eau.

19. Composition selon l'une quelconque des revendications précédentes, **caractérisée par le fait qu'**elle comprend un actif choisi parmi les actifs agissant sur la microcirculation, les actifs raffermissants et/ou anti-glycants, et leurs mélanges.

20. Procédé cosmétique pour lutter contre la cellulite et/ou la peau d'orange et/ou amincir la silhouette, comprenant l'application topique sur la peau d'une composition selon l'une quelconque des revendications précédentes.

## Claims

1. Composition comprising, in a physiologically acceptable medium, at least one xanthine base or a plant extract containing it, at least one polyurethane powder, and at least one non-ionic dimethicone copolyol.

2. Composition according to the preceding claim, **characterized in that** the xanthine base is chosen from: caffeine, theophylline, theobromine, acefylline, xanthinol nicotinate, diniprophylline, diprophylline, etamiphylline and its derivatives, etophylline, proxyphylline, pentophylline, propentophylline, pyridophylline and bamiphylline.

3. Composition according to either one of the preceding claims, **characterized in that** the xanthine base is chosen from caffeine, theophylline, theobromine and acefylline.

4. Composition according to the preceding claim, **characterized in that** said plant extract is chosen from extracts of tea, of coffee, of guarana, of maté and of cola.

5. Composition according to any one of the preceding claims, **characterized in that** the xanthine base is present in an amount ranging from 0.01% to 10% by weight, relative to the total weight of the composition, preferably ranging from 0.1% to 7% by weight, and preferentially from 0.1% to 3% by weight.

6. Composition according to any one of the preceding claims, **characterized in that** the polyurethane powder is not film-forming.

7. Composition according to any one of the preceding claims, **characterized in that** the polyurethane powder is a powder of a copolymer of hexamethylene diisocyanate and of trimethylol hexyl lactone.

8. Composition according to any one of the preceding claims, **characterized in that** it comprises a polyurethane powder in an amount ranging from 0.5% to 10% by weight, relative to the total weight of the composition, and preferably ranging from 0.5% to 6% by weight, and preferentially ranging from 0.5% to 3% by weight.

9. Composition according to any one of the preceding claims, **characterized in that** the non-ionic dimethicone copolyol is an oxypropylenated and/or oxyethylenated polydimethylmethylsiloxane that does not contain an ionic group.

10. Composition according to any one of the preceding claims, **characterized in that** the non-ionic dimethicone copolyol is present in an amount ranging from 0.05% to 5% by weight, relative to the total weight of the composition, preferably ranging from 0.1% to 3% by weight, and preferentially ranging from 0.1% to 2% by weight.

11. Composition according to any one of the preceding claims, **characterized in that** it comprises an aqueous phase.

12. Composition according to the preceding claim, **characterized in that** it comprises water in an amount ranging from 30% to 80% by weight, relative to the total weight of the composition, preferably ranging from 40% to 70% by weight, and preferentially ranging from 45% to 65% by weight.

13. Composition according to either one of Claims 11 and 12, **characterized in that** it comprises a monoalcohol having from 2 to 6 carbon atoms.

14. Composition according to the preceding claim, **characterized in that** the monoalcohol is chosen from ethanol and isopropanol.

15. Composition according to Claim 13 or 14, **characterized in that** the monoalcohol is present in an amount ranging from 1 to 30% by weight, relative to the total weight of the composition, and preferably ranging from 5% to 20% by weight.

16. Composition according to any one of Claims 12 to 15, **characterized in that** it comprises salicylic acid or one of its derivatives.

17. Composition according to the preceding claim, **characterized in that** the salicylic acid or its derivatives is present in an amount ranging from 0.1% to 5% by weight, relative to the total weight of the composition, preferably ranging from 0.1 to 3% by weight and preferentially ranging from 0.1% to 2% by weight.

18. Composition according to any one of the preceding claims, **characterized in that** it is in the form of an oil-in-water emulsion.

19. Composition according to any one of the preceding claims, **characterized in that** it comprises an active agent chosen from active agents that act on the microcirculation, firming and/or anti-glycant active agents, and mixtures thereof.

20. Cosmetic method for combating cellulite and/or "orange-peel" skin and/or slimming the figure, comprising the topical application to the skin of a composition according to any one of the preceding claims.

## Patentansprüche

1. Zusammensetzung, die in einem physiologisch akzeptablen Medium mindestens eine auf Xanthin basierende Verbindung oder einen sie enthaltenden Pflanzenextrakt, mindestens ein Polyurethanpulver und mindestens ein nichtionisches Dimethiconcopolyol enthält.

2. Zusammensetzung nach dem vorhergehenden Anspruch, **dadurch gekennzeichnet, dass** die auf Xanthin basierende Verbindung ausgewählt ist unter: Coffein, Theophyllin, Theobromin, Acefyllin, Xanthinolnicotinat, Diniprophyllin, Diprophyllin, Etamiphyllin und seinen Derivaten, Etophyllin, Proxyphyllin, Pentophyllin, Propentophyllin, Pyridophyllin und Bamiphyllin.

3. Zusammensetzung nach einem der vorhergehenden Ansprüche, **dadurch gekennzeichnet, dass** die Xanthinbase unter Coffein, Theophyllin, Theobromin und Acefyllin ausgewählt ist.

4. Zusammensetzung nach dem vorhergehenden Anspruch, **dadurch gekennzeichnet, dass** der Pflanzenextrakt unter den Extrakten von Tee, Kaffee, Guarana, Mate und Colanuss ausgewählt ist.

5. Zusammensetzung nach einem der vorhergehenden Ansprüche, **dadurch gekennzeichnet, dass** die auf Xanthin basierende Verbindung in einer Menge von 0,01 bis 10 Gew.-%, bezogen auf das Gesamtgewicht der Zusammensetzung, vorzugsweise 0,1 bis 7 Gew.-% und bevorzugt 0,1, bis 3 Gew.-% enthalten ist.

6. Zusammensetzung nach einem der vorhergehenden Ansprüche, **dadurch gekennzeichnet, dass** das Polyurethanpulver nicht filmbildend ist.

7. Zusammensetzung nach einem der vorhergehenden Ansprüche, **dadurch gekennzeichnet, dass** das Polyurethanpulver ein Pulver aus Hexamethylendiisocyaxlat/Trimethylolhexyllacton-Copolymer ist.

8. Zusammensetzung nach einem der vorhergehenden Ansprüche, **dadurch gekennzeichnet, dass** sie ein Polyurethanpulver in einer Menge von 0,5 bis 10 Gew.-%, bezogen auf das Gesamtgewicht der Zusammensetzung, vorzugsweise 0,5 bis 6 Gew.-% und bevorzugt 0,5 bis 3 Gew.-% enthält.

9. Zusammensetzung nach einem der vorhergehenden Ansprüche, **dadurch gekennzeichnet, dass** das nichtionische Dimethiconcopolyol ein propoxyliertes und/oder ethoxyliertes Polydimethylmethylsiloxan ist, das keine ionische Gruppe enthält.

10. Zusammensetzung nach einem der vorhergehenden Ansprüche, **dadurch gekennzeichnet, dass** das nichtionische Dimethiconcopolyol in einer Menge von 0,05 bis 5 Gew.-%, bezogen auf das Gesamtgewicht der Zusammensetzung, vorzugsweise 0,1 bis 3 Gew.-% und bevorzugt 0,1 bis 2 Gew.-% enthalten ist.

11. Zusammensetzung nach einem der vorhergehenden Ansprüche, **dadurch gekennzeichnet, dass** sie eine wässrige Phase enthält.

12. Zusammensetzung nach dem vorhergehenden Anspruch, **dadurch gekennzeichnet, dass** sie Wasser in einer Menge von 30 bis 80 Gew.-%, bezogen auf das Gesamtgewicht der Zusammensetzung, vorzugsweise 40 bis 70 Gew.-% und bevorzugt 45 bis 65 Gew.-% enthält.

13. Zusammensetzung nach einem der Ansprüche 11 oder 12, **dadurch gekennzeichnet, dass** sie einen Monoalkohol mit 2 bis 6 Kohlenstoffatomen enthält.

14. Zusammensetzung nach dem vorhergehenden Anspruch, **dadurch gekennzeichnet, dass** der Monoalkohol unter Ethanol und Isopropanol ausgewählt ist.

15. Zusammensetzung nach Anspruch 13 oder 14, **dadurch gekennzeichnet, dass** der Monoalkohol in einer Menge von 1 bis 30 Gew.-%, bezogen auf das Gesamtgewicht der Zusammensetzung, und vorzugsweise 5 bis 20 Gew.-% enthalten ist.

16. Zusammensetzung nach einem der Ansprüche 12 bis 15, **dadurch gekennzeichnet, dass** sie Salicylsäure oder ein Salicylsäurederivat enthält.

17. Zusammensetzung nach dem vorhergehenden Anspruch, **dadurch gekennzeichnet, dass** die Salicylsäure oder ihre Derivate in einem Mengenanteil von 0,1 bis 5 Gew.-%, bezogen auf das Gesamtgewicht der Zusammensetzung, vorzugsweise 0,1 bis 3 Gew.-% und bevorzugt 0,1 bis 2 Gew.-% enthalten sind.

18. Zusammensetzung nach einem der vorhergehenden Ansprüche, **dadurch gekennzeichnet, dass** sie als Öl-in-Wasser-Emulsion vorliegt.

19. Zusammensetzung nach einem der vorhergehenden Ansprüche, **dadurch gekennzeichnet, dass** sie einen Wirkstoff enthält, der unter den Wirkstoffen, die auf die Mikrozirkulation einwirken, straffenden Wirkstoffen und/oder Antiglycationswirkstoffen und deren Gemischen ausgewählt ist.

20. Kosmetisches Verfahren, um Cellulitis und/oder Orangenhaut zu bekämpfen und/oder die Silhouette schlanker zu machen, das das topische Auftragen einer Zusammensetzung nach einem der vorhergehenden Ansprüche auf die Haut umfasst.
